# EUROPEAN PATENT APPLICATION

(11) **EP 4 628 572 A1**
(43) Date of publication of application: **08.10.2025**
(21) Application number: 23903189.1
(22) Date of filing: 14.11.2023
(51) Int. Cl.: C12N 5/071, C12N 5/12

(54) **ULTRASMALL CELL CULTURING METHOD**

(30) Priority: 15.12.2022 JP 2022200443
(71) Applicant: Yamaha Hatsudoki Kabushiki Kaisha, Iwata-shi, Shizuoka 438-8501 (JP)
(72) Inventor: HARADA, Gakuro, Iwata-shi, Shizuoka 438-8501 (JP); HIKICHI, Yuichi, Iwata-shi, Shizuoka 438-8501 (JP); TSUJIOKA, Kazuya, Osaka-shi, Osaka 536-8523 (JP); TAHARA, Hiroshi, Osaka-shi, Osaka 536-8523 (JP)
(74) Representative: Müller-Boré & Partner Patentanwälte PartG mbB
(86) International application number: PCT/JP2023/041001
(87) International publication number: WO 2024/127891

(57) **Abstract**

A method for culturing an extremely small cell includes the steps of: inputting in a container including a plurality of storage sections each having an opening on an upper surface, a culture medium, in an amount in which a liquid level is located above the opening, and a plurality of single cells, and retaining the single cells in at least some of the plurality of storage sections; removing the culture medium in the container until the liquid level of the culture medium substantially coincides with a height position of the opening of each of the storage sections; and injecting a sealing liquid for preventing evaporation of the culture medium into the container to seal an upper part of the opening.

## Description

### Technical Field

The present invention relates to a method for culturing an extremely small cell by seeding an extremely small number of cells with respect to a culture area.

### Background Art

It may be necessary to culture in an extremely small culture environment in which only a small number of single cells or single origin cells are seeded and cultured with respect to the culture area, which is the region where the cells are cultured. For the development of an antibody drug having an effect of specifically binding to an antigen of a foreign substance such as a virus-infected cell or a cancer cell and removing the foreign substance, it is essential to culture cells capable of producing an antibody. The culture of the antibody-producing cells is often performed in the extremely small culture environment. There are various prior art methods for culturing cells. For example, Patent Literature 1 discloses a culturing method using alginic acid gel as a spheroid culture technique.

Screening for identifying cells having a high antibody production amount from among the cultured cells is required. Patent Literature 2 discloses a screening method in which cells cultured in a plurality of wells are irradiated with light, and the antibody production amount is evaluated based on the amount of fluorescence generated from the wells. As a next step of screening, an operation of culturing and proliferating cells having a high antibody production amount and producing a large amount of antibody is performed. However, there are many cells that are difficult to proliferate even if the antibody production amount is large.

### Citation List

### Patent Literature

Patent Literature 1: JP 2021-511078 A
Patent Literature 2: JP 6461580 B2

### Summary of Invention

An object of the present invention is to provide a culturing method capable of efficiently proliferating a single cell, such as an antibody-producing cell, which needs to be cultured in an extremely small culture environment.

A method for culturing an extremely small cell according to an aspect of the present invention includes the steps of: inputting in a container including a plurality of storage sections each having an opening on an upper surface, a culture medium, in an amount in which a liquid level is located above the opening, and a plurality of single cells, and retaining the single cells in at least some of the plurality of storage sections; removing the culture medium in the container until the liquid level of the culture medium substantially coincides with a height position of the opening of each of the storage sections; and injecting a sealing liquid for preventing evaporation of the culture medium into the container to seal an upper part of the opening.

### Brief Description of Drawings

FIG. 1 is a view showing a step flow of a method for culturing highly antibody-producing cells according to an embodiment of the present invention.
FIG. 2A is a plan view with an enlarged view showing a structure of a culture plate.
FIG. 2B is a cross-sectional view taken along line IIB-IIB of FIG. 2A.
FIG. 3 is a schematic cross-sectional view showing a step of seeding a single cell from a suction tip to a culture plate and retaining the single cell in a microgrid.
FIG. 4 is a schematic cross-sectional view showing a step of establishing an extremely small culture environment by culture medium suction.
FIG. 5 is a schematic cross-sectional view showing a situation in which an opening of a microgrid is sealed with a sealing liquid.
FIG. 6 is an image showing a proliferation situation of a single cell during a culture period in a culture plate.
FIG. 7 is an image of a proliferation situation of a single cell using a culturing method of a comparative example.
FIG. 8 is an image of a proliferation situation of a single cell using a culturing method of a comparative example.

### Description of Embodiments

Hereinafter, an embodiment of a method for culturing an extremely small cell according to the present invention will be described in detail with reference to the drawings. The culturing method of the present invention is directed to culture in an extremely small culture environment in which only a small number of single cells are seeded with respect to the culture area of the cells. The term "single cell" as used herein also includes a single origin cell which is a cell in which a single cell is proliferated. In general, single cells tend to be difficult to culture in the extremely small culture environment, and the present invention makes it possible to efficiently culture single cells in such an environment. Examples of the single cells include single cells capable of producing a recombinant protein, for example, single cells expected to be used in antibody drug production or antibody production, such as CHO cells and B cells. Hereinafter, an example in which antibody-producing cells are cultured in the extremely small culture environment will be described.

### [Overall flow of culture step]

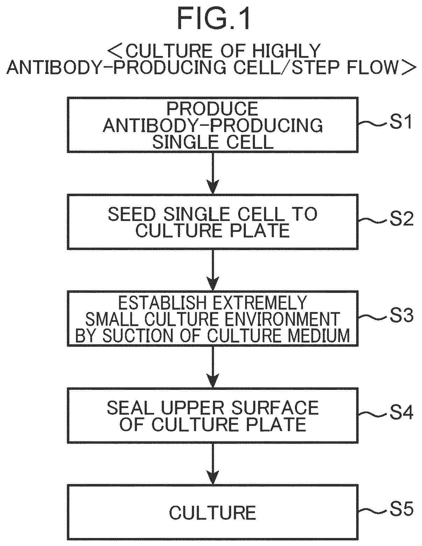

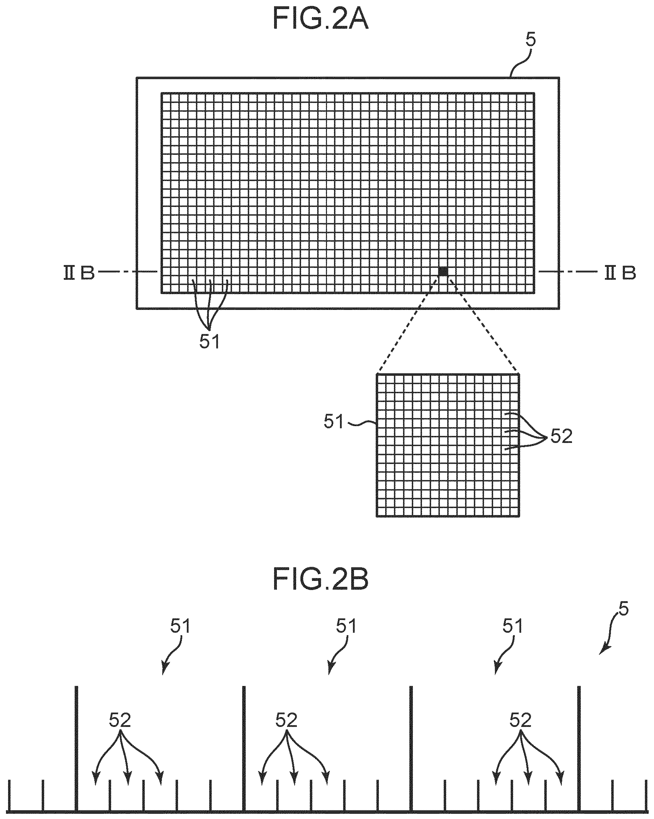

First, the overall flow of the method for culturing antibody-producing cells according to the present embodiment is described with reference to the step flow shown in FIG. 1. The culturing method of the present embodiment includes steps S1 to S5 sequentially performed. First, a large number of single cells capable of producing an antibody is produced using a predetermined method (step S1). Next, the produced single cells are seeded on a culture plate 5 (FIG. 2A and FIG. 2B) having a plurality of cell storage sections together with a liquid culture medium (step S2).

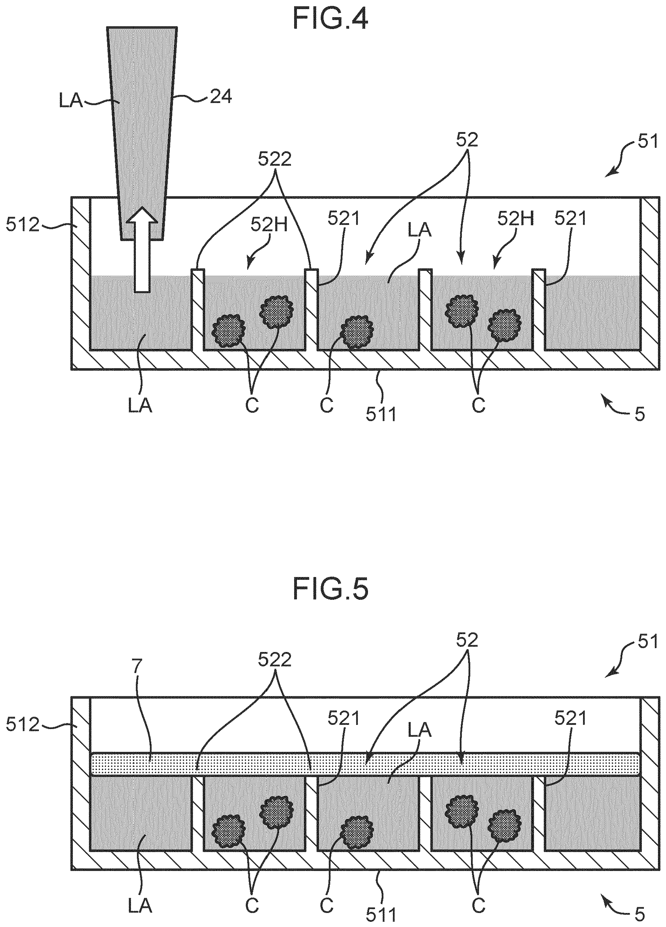

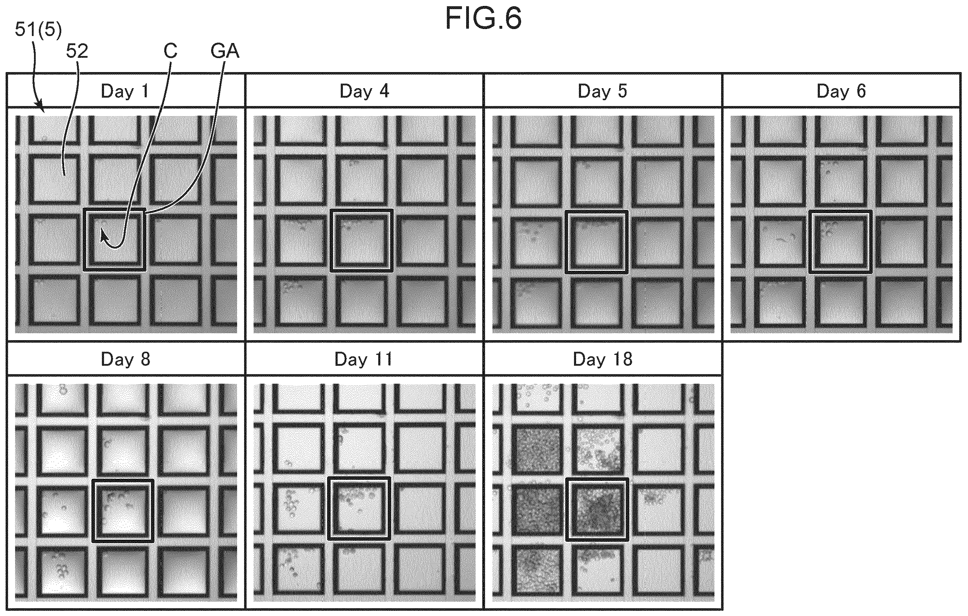

Subsequently, the liquid culture medium is suctioned from the culture plate 5 to establish an extremely small culture environment in which single cells are cultured in individual cell storage sections (step S3/FIG. 4). Further, the upper surface of the culture plate 5 is sealed with a sealing liquid for preventing evaporation of the culture medium (step S4/FIG. 5). Thereafter, single cells are cultured in the cell storage section for a predetermined number of days (step S5/FIG. 6). Hereinafter, each of steps S1 to S5 described above will be described in detail.

### [Step S1; Production of antibody-producing single cell]

In step S1, for example, by introducing a predetermined gene into a single cell to be cultured, the antibody producing ability is imparted to the single cell. A B cell of an immune cell can be exemplified as the single cell, and a monoclonal antibody produced by a single type of B cell can be exemplified as the antibody to be produced. The gene can be introduced by, for example, a chemical method using a carrier molecule such as a cationic lipid, a physical method such as electroporation, or a biological method such as a viral vector.

The single cells into which the gene have been introduced may be directly used as an object to be seeded in the next step S2, or a cell strain having a high antibody producing ability may be selected from the single cells and used as an object to be seeded. In this case, the single cell produced in step S1 is cultured in a microplate or the like for a predetermined period of time to give an antibody production period. Thereafter, a liquid culture medium containing a detection antibody that binds to the antibody produced by the single cell is added to the microplate, and a single cell having high antibody producing ability is identified. A single cell identified as a highly antibody-producing cell strain is picked with a micropipette or the like to prepare a cell suspension, and is seeded in step S2.

### [Step S2; Cell seeding on culture plate]

In step S2, the single cells produced in step S1 are seeded on a culture plate for culturing. FIG. 2A is a plan view with an enlarged view showing the structure of the culture plate 5, and FIG. 2B is a cross-sectional view taken along line IIB-IIB of FIG. 2A. The culture plate 5 includes a grid 51 formed of recesses arranged in a matrix on one surface of a flat plate-shaped base material, and a microgrid 52 formed of recesses of minute sizes arranged in a matrix in each grid 51.

The grid 51 is a large partitioned part that divides a relatively large-sized region of the culture plate 5. FIG. 2A illustrates a rectangular grid 51 partitioned by vertical and horizontal grid plates in top view. Instead of this, a structure in which the circular well-type grids 51 in top view are arranged in a honeycomb shape or a matrix shape may be adopted. The microgrid 52 is a small partitioned part that further subdivides the inside of each of the grids 51. The microgrid 52 is formed on a bottom plate of the grid 51, and is a recess that is rectangular in top view and is partitioned by a side plate lower than a grid plate partitioning the grid 51. The microgrid 52 may also be a circular well type in top view.

The microgrid 52 has an opening on the upper surface and serves as a storage section for retaining a single cell. As an example of the size of the microgrid 52, one side is 200 µm and the depth is 100 µm. The culture plate 5 is a plate having a plurality of storage sections partitioned in minute sizes. The microgrid 52 is desirably set to a size capable of forming a minute culture space, and can be set to, for example, a size selected from a range of an opening area of 4.0 × 10⁻² to 1.0 × 10⁻¹ mm² and a volume of 4.0 × 10⁻³ to 1.0 × 10⁻² mm³, more desirably a size selected from a range of an opening area of 1.0 × 10⁻³ to 1.0 × 10⁻¹ mm² and a volume of 2.0 × 10⁻⁵ to 1.0 × 10⁻² mm³.

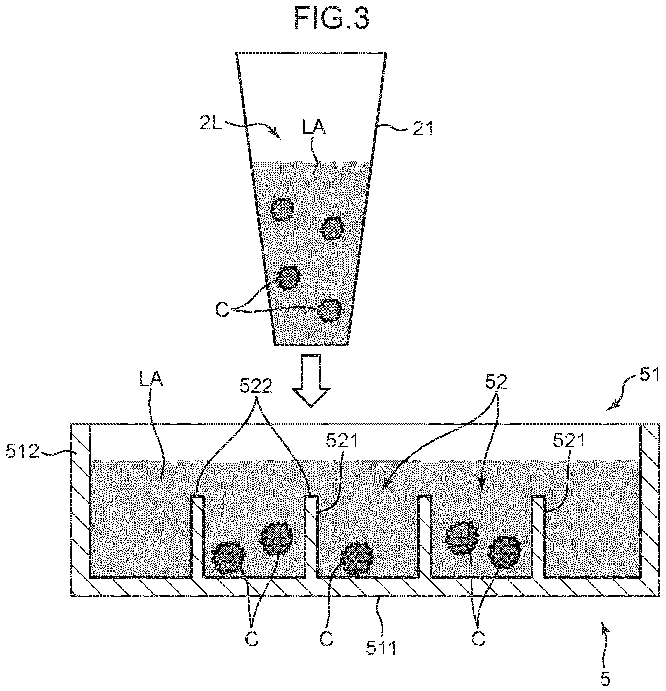

FIG. 3 is a schematic cross-sectional view showing a situation in which a single cell C is seeded on the culture plate 5. FIG. 3 shows a cross-sectional view of one grid 51 of the culture plate 5 shown in FIG. 2B. The grid 51 includes a grid bottom plate 511 forming a bottom surface and a grid side plate 512 forming a side surface. Each microgrid 52 is partitioned by a common grid bottom plate 511 and side plates 521 forming individual side surfaces.

At the time of seeding, the cell suspension 2L is prepared in which single cells C, prepared in step S1, capable of producing an antibody are contained in a liquid culture medium LA. The cell suspension 2L is stored in the dispensing container 21 and injected into each grid 51 of the culture plate 5. By this injection, the liquid culture medium LA and one or a plurality of single cells C are retained in at least some of the large number of microgrids 52 in the grid 51. Since the degree of dilution of the single cells C by the cell suspension 2L is an extremely small culture environment, for example, it can be set to such an extent that one single cell C is allocated to 20 to 25 microgrids 52.

Prior to the seeding of the single cells C, a predetermined amount of the liquid culture medium LA is inputted into the culture plate 5. As illustrated in FIG. 3, the input amount of the liquid culture medium LA is an amount by which the liquid level reaches above the top part 522 of the side plate 521 partitioning the microgrid 52. In other words, the amount of the liquid culture medium LA whose liquid level is located above the upper surface opening of the microgrid 52 is injected into the grid 51 of the culture plate 5 in advance. Note that the top parts 522 of the microgrids 52 are at the same height position. As the liquid culture medium LA, a normal culture solution containing a growth factor such as an inorganic salt, glucose, or amino acid and an additive component such as an antibiotic or a growth promoting factor in an aqueous medium may be used. For example, a CH150 culture medium (trade name of Gmep Incorporated) can be suitably used as the liquid culture medium LA.

Thereafter, the single cells C in the cell suspension 2L are seeded from the dispensing container 21 to the culture plate 5. By this seeding, among the plurality of the microgrids 52 included in one grid 51, one or a plurality of single cells C are retained in at least some of the microgrids 52.

### [Step S3; Establishment of extremely small culture environment by culture medium suction]

Step S3 is a step of suctioning the liquid culture medium LA of the culture plate 5 to establish a culture environment of an independent single cell C in units of the microgrids 52. The culture environment established here is a culture environment having an extremely small culture area. FIG. 4 is a schematic cross-sectional view showing the operation of step S3. FIG. 4 shows a state in which the liquid culture medium LA in the grid 51 is suctioned by a liquid absorbing tip 24.

The suction by the liquid absorbing tip 24 is performed from the state of FIG. 3 until the liquid level of the liquid culture medium LA of the grid 51 is in a state where the top part 522 of the side plate 521 of the microgrid 52 is exposed. That is, the liquid culture medium LA of the grid 51 is removed until the liquid level of the liquid culture medium LA substantially coincides with the height position of an opening 52H of the microgrid 52. By such suction, the liquid culture medium LA in one microgrid 52 and the liquid culture medium LA in the other microgrid 52 are not mixed. That is, an extremely small culture environment consisting of the liquid culture medium LA in the individual microgrids 52 for the single cell C is formed. The amount of the liquid culture medium LA in one microgrid 52 is, for example, 4 nanoliter.

In an extremely small culture environment in which a small number of single cells C of about 1 to 10 are input into a vast culture environment, the single cells C are difficult to proliferate. For example, in FIG. 4, even if the liquid culture medium LA is injected into the grid 51 in which the side plate 521 is removed and the partition of the microgrid 52 is eliminated, and the single cell C is input and given a predetermined culture period, the single cell C hardly proliferates. On the other hand, when about 1 to 10 single cells C are input and cultured in an extremely small culture environment in which the liquid culture medium LA is about 4 nanoliters, the cells are likely to be adjacent to each other, and the proliferation of the single cells C tends to be promoted. In the cell seeding in step S2, it is advantageous that the liquid level of the liquid culture medium LA is higher than the opening 52H because the single cell C can be retained in the microgrid 52 in one ejection operation. By suctioning the culture medium in the subsequent step S3, it is possible to establish an extremely small culture environment suitable for culture and proliferation of a small number of single cells C isolated in units of the microgrids 52.

### [Step S4; Sealing of microgrid]

Step S4 is a step of injecting a sealing liquid 7 into the culture plate 5 to seal the upper side of the opening 52H of the microgrid 52. FIG. 5 is a schematic cross-sectional view showing a situation where the opening 52H of the microgrid 52 is sealed with the sealing liquid 7. The lower surface of the sealing liquid 7 is in contact with the top part 522 of the side plate 521 of the microgrid 52 and closes the opening part 52H. That is, the liquid culture medium LA and the single cell C are confined in one microgrid 52 by the sealing liquid 7. As the sealing liquid 7, for example, an embryo culture oil made of light liquid paraffin or the like can be used.

The function required as the sealing liquid 7 is an evaporation preventing function of the liquid culture medium LA in the microgrid 52. Since the liquid culture medium LA contains moisture, the moisture evaporates when the sealing liquid 7 is not present. For this reason, during the culture in step S5, the amount of the liquid culture medium LA in the microgrid 52 decreases or is depleted, or a failure such as a change in the culture medium state such as osmotic pressure or pH occurs. By sealing the opening 52H with the sealing liquid 7 having an evaporation preventing function, evaporation of the liquid culture medium LA during the culture period can be suppressed.

Another desirable function of the sealing liquid 7 is air permeability. With the sealing liquid 7 having air permeability, even if the opening 52H of the microgrid 52 is sealed, the liquid culture medium LA in the microgrid 52 can be communicated with the atmosphere. Therefore, the culture environment of the single cell C in the microgrid 52 can be maintained healthy. The embryo culture oil described above has both the evaporation preventing function and the air permeability, and thus is suitable as the sealing liquid 7. Other than the embryo culture oil, other liquid or semi-liquid (gel) having at least an evaporation preventing function may be used as the sealing liquid 7. Needless to say, it is necessary that the specific gravity is smaller than that of the liquid culture medium LA.

By forming the layer of the sealing liquid 7, the extremely small culture environment established in step S3 can be maintained during the culture period. That is, not only evaporation of the liquid culture medium LA in the microgrid 52 can be prevented, but also contamination of foreign substance contained in the outside air, for example, minute dust, mold spores, bacteria, and the like into the microgrid 52 can be suppressed. There is also an advantage that diffusion of the active substance emitted by the single cell C cultured in the microgrid 52 can be prevented and the proliferation of the single cell C can be promoted.

### [Step S5; Cell culture]

Step S5 is a step of culturing the single cell C for a predetermined culture period in a state where the opening 52H of the microgrid 52 is sealed with the sealing liquid 7 as illustrated in FIG. 5. That is, it is a step of producing an antibody by culturing the single cell C produced in step S1 for a predetermined period and proliferating the single cell C. During this culture, a liquid culture medium containing growth factors is supplemented in the grid 51.

FIG. 6 is an image showing the proliferation situation of the single cell C during the culture period in the culture plate 5. "Day 1" in the figure means a state on the first day from the start of the secondary culture. FIG. 6 shows images of a part of the microgrid 52 included in one grid 51 on Day 1, Day 4, Day 5, Day 6, Day 8, Day 11, and Day 18 from the start of the culture. When the mutation situation of the single cell C in the target grid GA among the plurality of the microgrids 52 is observed, it is found that the single cell C is proliferated every day. Incidentally, the reason why the single cells C around the target grid GA also proliferate rapidly between Day 11 and Day 18 is that the number of culture days is simply long, and in addition, the single cells C proliferated from the target grid GA entered the adjacent grid when the liquid culture medium is supplemented.

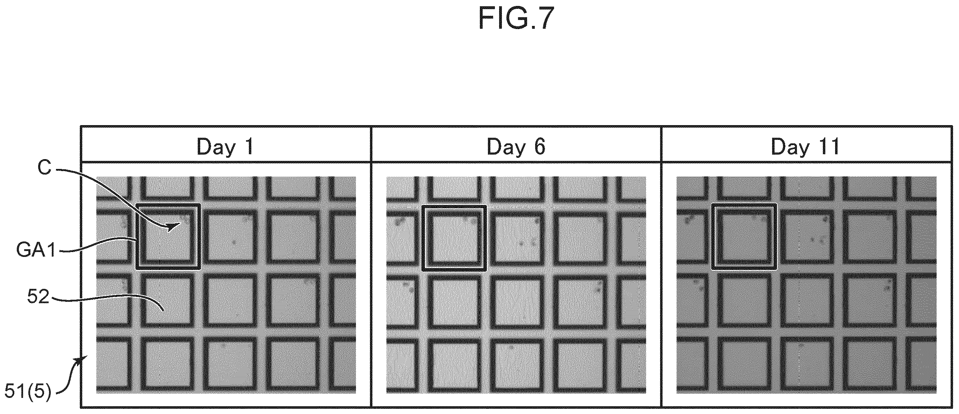

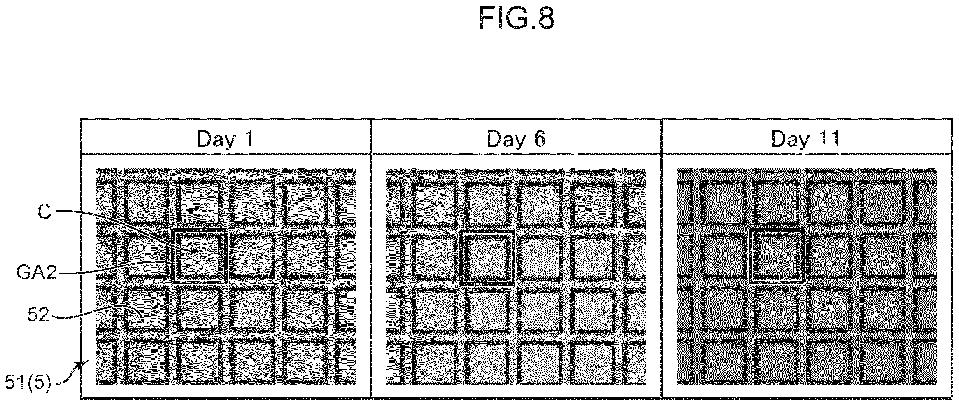

FIG. 7 and FIG. 8 are images of the proliferation situation of single cells using the culturing method of the comparative example. FIG. 7 is an image of Comparative Example 1 in which culture is performed immediately after a state in which the culture medium suction in step S3 (FIG. 4) is not performed and sealing with the embryo culture oil in step S4 (FIG. 5) is not performed, that is, after cell seeding in step S2 (FIG. 3). In FIG. 7, images on Day 1, Day 6, and Day 11 from the start of the culture are shown. Looking at the proliferation situation of the single cell C in the target grid GA1 among the plurality of the microgrids 52, it can be seen that no significant proliferation occurred between Day 1 and Day 11.

FIG. 8 is an image of Comparative Example 2 in which the culture was performed by injecting the sealing liquid 7 in a state in which the sealing with the embryo culture oil in step S4 was performed without performing the culture medium suction in step S3 (FIG. 4), that is, in a state in which the liquid level of the liquid culture medium LA is at a position higher than the top part 522 after the cell seeding in step S2 (FIG. 3). FIG. 8 also shows images on Day 1, Day 6, and Day 11 from the start of the culture. Looking at the proliferation situation of the single cell C in the target grid GA2 among the plurality of the microgrids 52, it can be seen that no significant proliferation occurred between Day 1 and Day 11.

### [Operation and effect]

The method for culturing an antibody-producing cell according to the present invention described above has the following operation and effect. That is, after the single cell C is retained by the microgrid 52 of the culture plate 5, the liquid culture medium LA is removed, and the opening 52H is sealed with the sealing liquid 7. This makes it possible to construct an extremely small culture environment closed in units of the microgrids 52 while preventing evaporation of the liquid culture medium LA in the microgrids 52. Culturing a single cell C in a small culture region promotes the proliferation of the single cell C. Therefore, by culturing the single cell C having an antibody producing ability as described above, the efficiency of proliferation of the single cell C can be improved, and eventually, a large amount of antibody can be produced.

### [Inventions included in the above embodiments]

The embodiment described above includes the invention having the following configuration.

A method for culturing an extremely small cell according to an aspect of the present invention includes the steps of: inputting in a container including a plurality of storage sections each having an opening on an upper surface, a culture medium, in an amount in which a liquid level is located above the opening, and a plurality of single cells, and retaining the single cells in at least some of the plurality of storage sections; removing the culture medium in the container until the liquid level of the culture medium substantially coincides with a height position of the opening of each of the storage sections; and injecting a sealing liquid for preventing evaporation of the culture medium into the container to seal an upper part of the opening.

According to this aspect, after the cells are retained in the storage section, the culture medium is removed and the opening is sealed with the sealing liquid, so that it is possible to construct a culture environment closed in units of storage sections while preventing evaporation of the culture medium in the storage section. It has been confirmed that culturing single cells in a small culture region promotes proliferation. Therefore, by culturing single cells as described above, the efficiency of proliferation can be improved even in an extremely small culture environment.

In the above culturing method, it is desirable that each of the single cells is a single cell capable of producing an antibody.

In the above culturing method, the step of retaining the single cell may include the steps of: injecting a culture medium in an amount in which a liquid level is located above the opening into the container in advance; and ejecting, to the container, the single cell picked with a tip together with a culture medium from another container.

According to this aspect, it is possible to sort and pick a single cell having high antibody producing ability in another container, retain the single cell in the storage section of the container, and culture and proliferate the single cell. That is, a cell strain having excellent antibody producing ability can be cultured in an environment in which the cell strain is more likely to proliferate.

In the above culturing method, it is desirable that the sealing liquid has air permeability. In this case, it is more desirable that the sealing liquid is an embryo culture oil.

According to this aspect, even when the opening of the storage section is sealed with the sealing liquid, the culture medium in the storage section can be communicated with the atmosphere, so that the culture environment in the storage section can be maintained soundly.

In the above culturing method, it is desirable that the storage section has a size selected from a range of an opening area of 1.0 × 10⁻³ to 1.0 × 10⁻¹ mm² and a volume of 2.0 × 10⁻⁵ to 1.0 × 10⁻² mm³.

According to this aspect, a culture region of single cells can be sufficiently miniaturized, and a large number of single cells can be independently cultured with a limited plate area. Therefore, the efficiency of culture and proliferation of single cells can be improved.

In the above culturing method, it is preferable that the container includes a large partitioned part that divides a relatively large-sized region of the container, and a small partitioned part that further subdivides an inside of the large partitioned part, and the small partitioned part is the storage section.

According to this aspect, it is possible to utilize it by, for example, changing a cell type or a culture solution in units of large partitioned parts, and culture can be diversified.

According to the present invention described above, it is possible to provide a culturing method capable of efficiently proliferating a single cell, such as an antibody-producing cell, which needs to be cultured in an extremely small culture environment.

## Claims

1. method for culturing an extremely small cell, the method comprising the steps of:
inputting in a container including a plurality of storage sections each having an opening on an upper surface, a culture medium, in an amount in which a liquid level is located above the opening, and a plurality of single cells, and retaining the single cells in at least some of the plurality of storage sections;
removing the culture medium in the container until the liquid level of the culture medium substantially coincides with a height position of the opening of each of the storage sections; and
injecting a sealing liquid for preventing evaporation of the culture medium into the container to seal an upper part of the opening.

2. The method for culturing an extremely small cell according to claim 1, wherein each of the single cells is a single cell capable of producing an antibody.

3. The method for culturing an extremely small cell according to claim 1 or 2, wherein
the step of retaining the single cell includes the steps of:
injecting a culture medium in an amount in which a liquid level is located above the opening into the container in advance; and
ejecting, to the container, the single cell picked with a tip together with a culture medium from another container.

4. The method for culturing an extremely small cell according to claim 1 or 2, wherein the sealing liquid has air permeability.

5. The method for culturing an extremely small cell according to claim 1 or 2, wherein the sealing liquid comprises an embryo culture oil.

6. The method for culturing an extremely small cell according to claim 1, wherein the storage section has a size selected from a range of an opening area of 1.0 × 10⁻³ to 1.0 × 10⁻¹ mm² and a volume of 2.0 × 10⁻⁵ to 1.0 × 10⁻² mm³.

7. The method for culturing an extremely small cell according to claim 6, wherein
the container includes a large partitioned part that divides a relatively large-sized region of the container, and a small partitioned part that further subdivides an inside of the large partitioned part, and
the small partitioned part is the storage section.
